# EUROPEAN PATENT APPLICATION

(11) **EP 1 621 611 A1**
(43) Date of publication of application: **01.02.2006**
(21) Application number: 04725186.3
(22) Date of filing: 01.04.2004
(51) Int. Cl.: C12N 5/06, C12N 15/12, C12Q 1/02, C12N 5/10

(54) **TONGUE EPITHELIUM-ORIGIN CELL LINE KT-1 AND USE THEREOF**

(30) Priority: 01.04.2003 JP 2003098516
(71) Applicant: National Food Research Institute, Tsukuba-shi, Ibaraki 305-8642 (JP); National Agriculture and Bio-oriented Research Organization, Ibaraki 305-0856 (JP)
(72) Inventor: OOKURA, Tetsuya, TSukuba-shi, Ibaraki 3050044 (JP); KAWAMOTO, Keiko, Obihiro-shi, Hokkaido 0802474 (JP); HINO, Akihiro, Tsukuba-shi, Ibaraki 3050031 (JP)
(74) Representative: Hallybone, Huw George
(86) International application number: PCT/JP2004/004788
(87) International publication number: WO 2004/090122

(57) **Abstract**

A cell line KT-1 isolated from mouse tongue epithelium using high-level expression of integrin β1 as the indicator and established by a long time culture, and which expresses a taste receptor; a taste sensor comprising the cell line KT-1; the use of the cell line KT-1 to search for a factor which induces differentiation of taste cells, a substance which controls expression of taste receptors, a novel taste substance, a taste-modifying substance and a substance which aids in taste-suppressed state.

SEQUENCE LISTING

<110> National Food Research Institute
National Agriculture and Bio-oriented Research Organization

<120> A cell line designated KT-1 established from tongue epithelial cells and use thereof

<130> Y1L-0254

<150> JP-P2003 98516
<151> 2003-04-01

<160> 34

<170> PatentIn version 3.1

<210> 1
<211> 20
<212> DNA
<213> Artificial

<220>
<223> primer

<400> 1
accgttgagg agataaacaa 20

<210> 2
<211> 20
<212> DNA
<213> Artificial

<220>
<223> primer

<400> 2

tcacccgcct gggcggagag                                                    20

<210>  3
<211>  20
<212>  DNA
<213>  Artificial

<220>
<223>  primer

<400>  3
gagcgcaaca cttcagctta                                                    20

<210>  4
<211>  20
<212>  DNA
<213>  Artificial

<220>
<223>  primer

<400>  4
cacccaaggg aaatttattc                                                    20

<210>  5
<211>  20
<212>  DNA
<213>  Artificial

<220>
<223>  primer

<400>  5
tggatgtact acgccaagtg                                                    20

<210> 6
<211> 22
<212> DNA
<213> Artificial

<220>
<223> primer

<400> 6
agcatccaga tgacttcacc tg 22

<210> 7
<211> 25
<212> DNA
<213> Artificial

<220>
<223> primer

<400> 7
ctgattgaaa tcatctctta tatat 25

<210> 8
<211> 25
<212> DNA
<213> Artificial

<220>
<223> primer

<400> 8
tctattgaaa cacctataaa ataca 25

<210> 9
<211> 20
<212> DNA
<213> Artificial

<220>
<223> primer

<400> 9
cctttcttcg tgttcctggc 20

<210> 10
<211> 27
<212> DNA
<213> Artificial

<220>
<223> primer

<400> 10
aagtctagaa gcagcagaat aggttgt 27

<210> 11
<211> 22
<212> DNA
<213> Artificial

<220>
<223> primer

<400> 11
catccctcac ccactctttc tg 22

<210> 12
<211> 23
<212> DNA
<213> Artificial

<220>
<223> primer

<400> 12
aagacctaga ctccatttgc cct 23

<210> 13
<211> 18
<212> DNA
<213> Artificial

<220>
<223> primer

<400> 13
ctcctctcac ccaggcca 18

<210> 14
<211> 23
<212> DNA
<213> Artificial

<220>
<223> primer

<400> 14
tgagtatctg cctacctggt cca 23

<210> 15

<211> 25
<212> DNA
<213> Artificial

<220>
<223> primer

<400> 15
tctgaactaa ttccacttcc catct 25

<210> 16
<211> 25
<212> DNA
<213> Artificial

<220>
<223> primer

<400> 16
tcaaagactg tgggctcttc tgagc 25

<210> 17
<211> 18
<212> DNA
<213> Artificial

<220>
<223> primer

<400> 17
cctgtccggt gtcatccg 18

<210> 18
<211> 21

<212> DNA
<213> Artificial

<220>
<223> primer

<400> 18
ggcggtatct ctcacgcttc t 21

<210> 19
<211> 22
<212> DNA
<213> Artificial

<220>
<223> primer

<400> 19
ggctttattg agaggatggc at 22

<210> 20
<211> 21
<212> DNA
<213> Artificial

<220>
<223> primer

<400> 20
aaggccacga agatggaaaa c 21

<210> 21
<211> 22
<212> DNA

<213>  Artificial

<220>
<223>  primer

<400>  21
gcccttgtca tctctgcttt tg                                                22

<210>  22
<211>  24
<212>  DNA
<213>  Artificial

<220>
<223>  primer

<400>  22
caccttacag agagtgtggg tcag                                              24

<210>  23
<211>  24
<212>  DNA
<213>  Artificial

<220>
<223>  primer

<400>  23
gaagccctgc catacctttc caga                                              24

<210>  24
<211>  24
<212>  DNA
<213>  Artificial

<220>
<223>  primer

<400>  24
ccagctacct gctggatatc gaca                                                24

<210>  25
<211>  20
<212>  DNA
<213>  Artificial

<220>
<223>  primer

<400>  25
catatttggg gccttcgctg                                                     20

<210>  26
<211>  20
<212>  DNA
<213>  Artificial

<220>
<223>  primer

<400>  26
caagcatcag taggtagccg                                                     20

<210>  27
<211>  22
<212>  DNA
<213>  Artificial

<220>
<223>  primer

<400>  27
aagaggagat ccgtgagttg ca                                                22

<210>  28
<211>  23
<212>  DNA
<213>  Artificial

<220>
<223>  primer

<400>  28
acaactgaat tgggtttgga tgg                                               23

<210>  29
<211>  18
<212>  DNA
<213>  Artificial

<220>
<223>  primer

<400>  29
acctggtgca gacccagc                                                     18

<210>  30
<211>  27
<212>  DNA
<213>  Artificial

<220>

<223>  primer

<400>  30
tgttcatatt ttacagacac acaggga                                          27

<210>  31
<211>  21
<212>  DNA
<213>  Artificial

<220>
<223>  primer

<400>  31
gcctctacag cttcttcggg a                                                21

<210>  32
<211>  26
<212>  DNA
<213>  Artificial

<220>
<223>  primer

<400>  32
tggatccttc tgtgtgctta tacatc                                           26

<210>  33
<211>  19
<212>  DNA
<213>  Artificial

<220>
<223>  primer

<400>  33
   taagccaaca agccccgaa                                                    19

<210>  34
<211>  20
<212>  DNA
<213>  Artificial

<220>
<223>  primer

<400>  34
ttgcggcaac ttttctgtcc                                                    20

## Description

### TECHNICAL FIELD

The present invention relates to a taste sensor and to a method for searching for the taste sensor. and the like using same, and relates more specifically to a cell line KT-1 established from mouse tongue epithelial cells, and to a use thereof. More particularly, it relates to a method of using the cell line KT-1 established from mouse tongue epithelial cells as a taste sensor, and to a method of using the cell line KT-1 to search for a factor which induces differentiation of taste cells, a substance which controls expression of taste receptors, a novel taste substance, a taste-modifying substance and a substance which aids in taste-suppressed state.

### BACKGROUND ART

Methods have already been reported of isolating and culturing tongue epithelial cells (see for example In Vitro Cell Dev. Biol. 38: 365-372, 2002) . However, there have been no reports of cell lines which are derived from tongue epithelium and express taste receptors, or cell lines comprising cell populationsat different stagesof differentiation. Asaconsequence, it has been extremely difficult to reproducibly measure specific taste responses using living cells, or to construct a system for identifying factors which induce taste cell differentiation.

### DISCLOSURE OF THE INVENTION

It is a first object of the present invention to provide a cell expressing a taste receptor, and a transformant thereof.

It is a second object of the present invention to provide a taste sensor using the aforementioned cell.

It is a third object of the present invention to provide a use for the aforementioned taste receptor.

It is a fourth object of the present invention to establish, using the cell at various stages of differentiation, a system for searching for a factor which induces differentiation to a specific taste cell and a substance which controls expression of taste receptors.

The present invention is explained in detail below.

### MODE FOR CARRYING OUT THE INVNTION

The present invention provides the following cells, taste sensor and uses therefor.
1. A cell line KT-1 isolated from mouse tongue epithelium using high-level expression of integrin β1 as the indicator and established by a long-term culture, and which expresses a taste receptor.
2. A cell line KT-1 established by a long-term culture of the cell line KT-1 described item 1 above in the following low-serum medium.

| | |
|---|---|
| Calcium | Final concentration 5 µg/ml |
| Epidermal growth factor | Final concentration 10 ng/ml |
| Basic fibroblast growth factor | Final concentration 10 ng/ml |
| Insulin | Final concentration 5 ng/ml |
| Transferrin | Final concentration 10 ng/ml |
| Hydrocortisone | Final concentration 0.2 µM |
| Ethanolamine | Final concentration 0.5 µM |
| Phosphoethanolamine | Final concentration 0.5 µM |
| Heparin | Final concentration 20 µg/ml |
| Gentamicin | Final concentration 10 mg/ml |
| Amphotericin B | Final concentration 500 ng/ml |
| Penicillin | Final concentration 50 U/ml |
| Streptomycin | Final concentration 50 mg/ml |
| Calcium-chelated fetal bovine serum | Final concentration 0.25% |

3. The cell line KT-1 according to item 2 above, deposited under Accession Number FERM BP-8347.
4. A KT-1 cell obtained by culturing the cell line KT-1 according to item 2 or 3 above in the following differentiation-inducing medium.

| | |
|---|---|
| Calcium | Final concentration 10 µg/ml |
| Epidermal growth factor | Final concentrationl ng/ml |
| Keratinocyte growth factor | Final concentrationl ng/ml |
| Insulin-like growth factor | Final concentration 5 ng/ml |
| Transferrin | Final concentration 100 ng/ml |
| Hydrocortisone | Final concentration 0.2 µM |
| Aldosterone | Final concentration 10 µM |
| Ethanolamine | Final concentration 0.5 µM |
| Phosphoethanolamine | Final concentration 0.5 µM |
| Calcitriol | Final concentration 10 nM |
| 9-cis retinoic acid | Final concentration 0.8 µM |
| Glutathione | Final concentration 200 µM |
| Cysteine | Final concentration 50 µM |
| Tyrosine | Final concentration 2.72 µg/ml |
| Phenylalanine | Final concentration 4.95 µg/ml |
| Heparin | Final concentration 20 µg/ml |
| Gentamicin | Final concentration 10 mg/ml |
| Amphotericin B | Final concentration 500 ng/ml |
| Penicillin | Final concentration 50 U/ml |
| Streptomycin | Final concentration 50 mg/ml |

5. The KT-1 cell according to item 4 above which functionally expresses a taste receptor.
6. A taste sensor comprising the cell line KT-1 according to any of items 1 through 3 above or the KT-1 cell according to item 4 or 5 above.
7. The use of a cell line KT-1 as a taste sensor, wherein, the cell line KT-1 according to any of items 1 through 3 above or the KT-1 cell according to item 4 or 5 above is used to distinguish a taste substance received by a taste receptor.
8. The use according to item 7 above, wherein the taste receptor is a sweet taste receptor, bitter taste receptor, salt taste channel or amino acid receptor.
9. A cell line KT-1 transformed by cDNA transfection of a taste receptor which is not expressed in cell line KT-1 and a ligand for which is known.
10. The use of the transformed cell line KT-1 according to item 9 above as a cell for detecting a specific taste which is received by the taste receptor.
11. A cell line KT-1 transformed by cDNA transfection of a taste receptor which is not expressed in cell line KT-1 and ligand for which is unknown.
12. A method for searching for an unknown ligand by using the transformed cell line KT-1 according to item 11 above.
13. The use of the cell line KT-1 according to any of items 1 through 3 above or the KT-1 cell according to item 4 or 5 above for searching for a factor which induces differentiation of taste cells, a substance which controls expression of taste receptors, a novel taste substance, a taste-modifying substance and a substance which aids in taste-suppressed state.
14. A method of searching for a taste receptor for a taste substance using the taste substance and the cell line KT-1 according to any of items 1 through 3 above or the KT-1 cell according to item 4 or 5 above.
15. The method according to item 14 above, wherein the taste receptor is a salt sensing channel.

The cell line KT-1 of the present invention consists of cells established by first isolating and culturing mouse tongue epithelial cells and then passaging and maintaining them for two-and-a-half years or more. Cell line KT-1 is a unique cell line consisting of a mixture of an undifferentiated cell and a cell expressing a taste receptor, and is deposited at the International Patent Organism Depository, National Institute of Advanced Industrial Science and Technology, Tsukuba Center 6, 1-1-1 Higashi, Tsukuba, Ibaraki, Post Code: 305-8566 as FERM BP-8347 (deposit date: March 27, 2003).

### BRIEF DESCRIPTION OF THE DRAWINGS

Figure 1 shows the results of RT-PCR using total RNA extracted from cell line KT-1A and specific primers.
Figure 2 shows results for detection of taste receptor mRNA in cell line KT-1A. In the figure, lane 1 is T1r1, lane 2 is T1r2, lane 3 is T1r3 and lane 4 is cytokeratin 8.
Figure 3 shows results for detection of taste receptor mRNA in KT-1C cells. In the figure, lane 1 is T1r1, lane 2 is T1r2, lane 3 is T1r3 and lane 4 is cytokeratin 8.
Figure 4 is an immunohistochemical staining showing the pattern of integrin expression in adult mouse circumvallate papilla.
Figure 5 shows the results of flow cytometric analysis of the level of expression of various integrin molecules in cell line KT-1B.
Figure 6 shows the results of immuno-staining and in situ hybridization for molecules expressed specifically in undifferentiated cells and taste bud cells of cell line KT-1B (top) and mouse circumvallate papilla (bottom).
Figure 7 shows results for calcium imaging of sweet taste receptor response in KT-1C cells.
Figure 8 shows hormone-induced changes in αENaC expression of cell line KT-1B. (Lane 1: progesterone, lane 2: hydrocortisone, lane 3: aldosterone).
Figure 9 shows results of sodium imaging for salt taste response in KT-1C cells.

### BEST MODE FOR CARRYING OUT THE INVENTION

The present invention is explained in more detail below using examples, but the present invention is not limited by the following examples.

### Examples

### 1. Method of establishing cell line KT-1

### 1-1. Establishment of cell line KT-1A

Cells were collected from mouse tongue epithelium using high-level expression of integrin β1 as the idnicator, and were maintained and cultured (once every 1 to 2 weeks) in the following medium for a year and a half after the primary culture.

### Medium: MCDB153 basic medium A

| | |
|---|---|
| Calcium | Final concentration 5 µg/ml |
| Epidermal growth factor | Final concentration 10 ng/ml |
| Basic fibroblast growth factor | Final concentration 10 ng/ml |
| Insulin | Final concentration 5 ng/ml |
| Transferrin | Final concentration 10 ng/ml |
| Hydrocortisone | Final concentration 0.2 µM |
| Ethanolamine | Final concentration 0.5 µM |
| Phosphoethanolamine | Final concentration 0.5 µM |
| Heparin | Final concentration 20 µg/ml |
| Gentamicin | Final concentration 10 mg/ml |
| Amphotericin B | Final concentration 500 ng/ml |
| Penicillin | Final concentration 50 U/ml |
| Streptomycin | Final concentration 50 mg/ml |
| Calcium-chelated fetal bovine serum | Final concentration 0.5% |

The resulting cell line was used as line KT-1A.

### 1-2. Establishment of cell line KT-1B

For purposes of passage, a plastic dish with cell line KT-1A adhering thereto was washed three times with PBS(-), and 0.05% trypsin-1 mM EDTA was added and reacted for 5 to 10 minutes at 37°C. Cells that peeled from the dish were collected by centrifugation after neutralization of trypsin activity, suspended in new medium, and seeded on a plastic dish coated with collagen type IV (3 µg/ml ) and Matrigel (10 µg/ml). They were then cultured once every 3 to 4 days continuously for a year and a half or more in the following medium, which has a lower serum concentration (0.25%).

### Medium: MCDB153 basic medium B

| | |
|---|---|
| Calcium | Final concentration 5 µg/ml |
| Epidermal growth factor | Final concentration 10 ng/ml |
| Basic fibroblast growth factor | Final concentration 10 ng/ml |
| Insulin | Final concentration 5 ng/ml |
| Transferrin | Final concentration 10 ng/ml |
| Hydrocortisone | Final concentration 0.2 µM |
| Ethanolamine | Final concentration 0.5 µM |
| Phosphoethanolamine | Final concentration 0.5 µM |
| Heparin | Final concentration 20 µg/ml |
| Gentamicin | Final concentration 10 mg/ml |
| Amphotericin B | Final concentration 500 ng/ml |
| Penicillin | Final concentration 50 U/ml |
| Streptomycin | Final concentration 50 mg/ml |
| Calcium-chelated fetal bovine serum | Final concentration 0.25% |

The resulting cell line was used as line KT-1B. Cell line KT-1B was deposited as FERM BP-8347.

### 1-3. Inducing differentiation of cell line KT-1B into KT-1C cells.

Cell line KT-1B was cultured for 3 to 7 days in the following differentiation-inducing medium to differentiate cell line KT-1B still further and obtain KT-1C cells.

### Differentiation-inducing medium: MCDB basic medium C

| | |
|---|---|
| Calcium | Final concentration 10 µg/ml |
| Epidermal growth factor | Final concentrationl ng/ml |
| Keratinocyte growth factor | Final concentrationl ng/ml |
| Insulin-like growth factor | Final concentration 5 ng/ml |
| Transferrin | Final concentration 100 ng/ml |
| Hydrocortisone | Final concentration 0.2 µM |
| Aldosterone | Final concentration 10 nM |
| Ethanolamine | Final concentration0.5 µM |
| Phosphoethanolamine | Final concentration 0.5 µM |
| Calcitriol | Final concentration 10 nM |
| 9-cis retinoic acid | Final concentration 0.8 µM |
| Glutathione | Final concentration 200 µM |
| Cysteine | Final concentration 50 µM |
| Tyrosine | Final concentration 2.72 µg/ml |
| Phenylalanine | Final concentration 4.95 µg/ml |
| Heparin | Final concentration 20 µg/ml |
| Gentamicin | Final concentration 10 mg/ml |
| Amphotericin B | Final concentration 500 ng/ml |
| Penicillin | Final concentration 50 U/ml |
| Streptomycin | Final concentration 50 mg/ml |

### 2-1. Confirmation of taste-associated gene mRNA expression in cell line KT-1A by RT-PCR

### (Experimental methods)

Cell line KT-1A cDNA was obtained by a reverse transcription reaction using as the template total RNA prepared from cell line KT-1A. A Trizol solution (Invitrogen) or the like can be easily used for RNA extraction. A reverse transcription reaction can be easily accomplished using 2 µg of this total RNA and a Super Script First Strand cDNA synthesis kit (Invitrogen).

Using this cDNA as the template and previously-designed primers specific to various taste receptors and taste bud-associated molecules (0.2 µM each, Table 1), 2.5 units of Takara Taq (Takara) were added and a PCR reaction was performed consisting of denaturation at 95°C for 5 minutes followed by 35 cycles of 1 minute at 95°C, 1 minute at 55-58°C and 2 minutes at 72°C in a 25 µl system. The various primer sequences are shown in Table 1 (however, the primers of Seq. Nos. 31 through 34 were used in example 2-2 as described below). 8 µl of the PCR reaction liquid was electrophoresed on a 1.5% agarose gel, stained with ethidium bromide and photographed.

**Table 1 Oligonucleotide primers, annealing temperatures(°C)and PCR product sizes (bp) for taste bud- and undifferentiated cell-specific markers.**

| | Primer 1 | Primer 2 | °C | Bp |
|---|---|---|---|---|
| T1r1 | Seq. No. 1 accgttgaggagataaacaa | Seq. No. 2 tcacccgcctgggcggagag | 58 | 524 |
| T1r2 | Seq. No. 3 gagcgcaacacttcagctta | Seq. No. 4 cacccaagggaaatttattc | 58 | 724 |
| T1r3 | Seq. No. 5 tggatgtactacgccaagtg | Seq. No.6 agcatccagatgacttcacctg | 56 | 859 |
| T2r5 | Seq. No. 7 ctgattgaaatcatctcttatatat | Seq. No. 8 tctattgaaacacctataaaataca | 58 | 503 |
| T2r18 | Seq. No. 9 cctttcttcgtgttcctggc | Seq. No. 10 aagtctagaagcagcagaataggttgt | 58 | 801 |
| T2r19 | Seq. No. 11 catccctcacccactctttctg | Seq. No. 12 aagacctagactccatttgccct | 58 | 839 |
| α ENaC | Seq. No. 13 ctcctctcacccaggcca | Seq. No. 14 tgagtatctgcctacctggtcca | 58 | 850 |
| Gustducin | Seq. No. 15 tctgaactaattccacttcccatct | Seq. No. 16 tcaaagactgtgggctcttctgagc | 58 | 1021 |
| Gαi2 | Seq. No.17 cctgtccggtgtcatccg | Seq. No. 18 ggcggtatctctcacgcttct | 58 | 801 |
| Gαi3 | Seq. No. 19 ggctttattgagaggatggcat | Seq. No. 20 aaggccacgaagatggaaaac | 58 | 852 |
| Gy13 | Seq. No. 21 gcccttgtcatctctgcttttg | Seq. No.22 caccttacagagagtgtgggtcag | 58 | 301 |
| STG | Seq. No. 23 gaagccctgccatacctttccaga | Seq. No. 24 ccagctacctgctggatatcgaca | 56 | 606 |
| Patched 1 | Seq. No. 25 catatttggggccttcgctg | Seq. No. 26 caagcatcagtaggtagccg | 58 | 1031 |
| Cytokeratin 8 | Seq. No. 27 aagaggagatccgtgagttgca | Seq. No. 28 acaactgaattgggtttggatgg | 58 | 942 |
| Notch 1 | Seq. No. 29 acctggtgcagacccagc | Seq. No. 30 tgttcatattttacagacacacaggga | 58 | 802 |
| T1r1 | Seq. No. 31 gcctctacagcttcttcggga | Seq. No. 32 tggatccttctgtgtgcttatacatc | 58 | 803 |
| T1r3 | Seq. No. 33 taagccaacaagccccgaa | Seq. No. 34 ttgcggcaacttttctgtcc | 58 | 662 |

### (Experimental results)

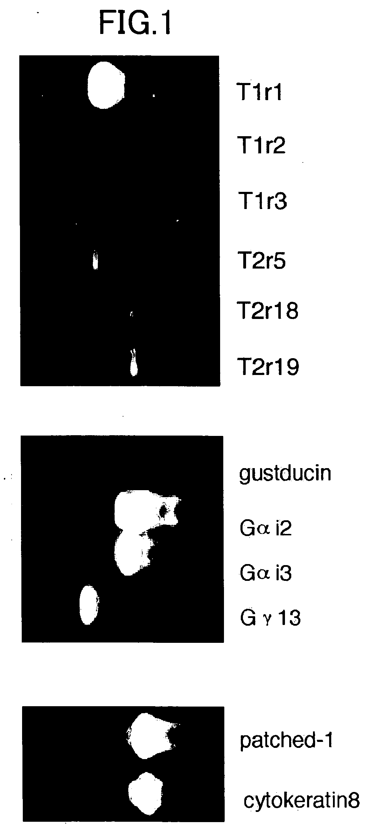

The results are shown in Figure 1. Expression of T1r1, T2r5, T2r18 and T2r19, which encode taste receptors, αENaC (amiloride-sensitive sodium channel alpha subunit) and the G proteins Gαi2, Gαi3 and Gγ13, which are involved in salt taste reception, and STG, cytokeratin 8 and Patched 1 (sometimes called Ptc1 orpatched-1 in the Figures) , which are specifically expressed in and around the taste buds, was confirmed in cell line KT-1A, but T1r2, T1r3 and gustducin were not expressed. From this it can be seen that cell line KT-1A consists of cells at a variety of stages of differentiation, from undifferentiated cells to cells expressing taste receptors.

### 2-2 Detection of taste receptor mRNA in cell line KT-1A and KT-1C cells

### (Experimental methods)

RNA was extracted from cell line KT-1A and KT-1C cells using Trizol solutions. cDNA was synthesized on a scale of 40 µl using 2 pg of the resulting RNA and a Super Script First Strand cDNA synthesis kit. Using 1 µl of this cDNA as the template and 0.2 µM each of primers (T1r1: Seq. Nos. 31 and 32, T1r3: Seq. Nos. 33 and 34, T1r2: Seq. Nos. 3 and 4, cytokeratin 8: Seq. Nos. 27 and 28) specific to various taste receptors (T1r1, T1r3, T1r2 and cytokeratin 8), 2.5 units of Takara Taq (Takara) were added and a PCR reaction was performed consisting of denaturation at 95°C for 5 minutes followed by 35 cycles of 1 minute at 95°C, 1 minute at 58-68°C and 2 minutes at 72°C in a 25 µl system. 8 µl of the PCR reaction liquid was electrophoresed on a 1.5% agarose gel, stained with ethidium bromide and photographed.

### (Experimental results)

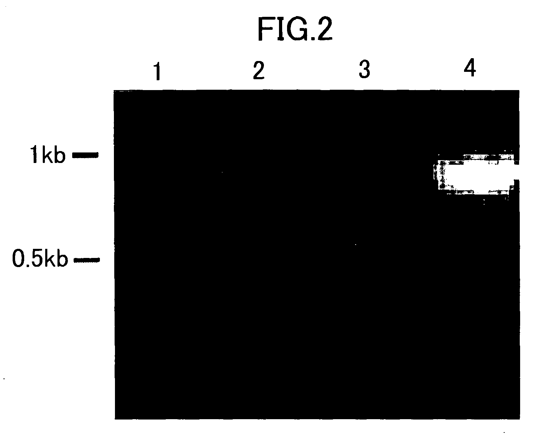

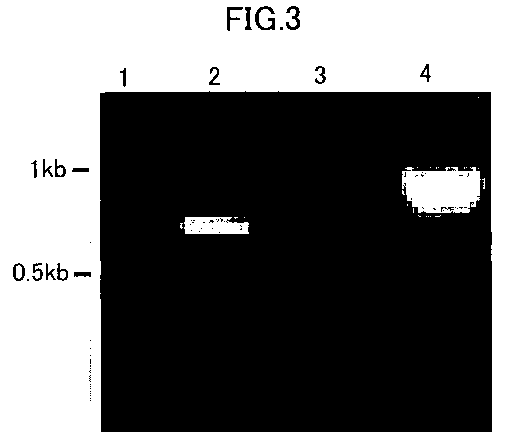

Figures 2 and 3 show results for detection of taste receptor mRNA in cell line KT-1A (Figure 2) and KT-1C cells (Figure 3) by RT-PCR. In both figures, lane 1 is T1r1, lane 2 is T1r2, lane 3 is T1r3 and lane 4 is cytokeratin 8.

Comparing Figures 2 and 3, sweet taste receptors T1r2 and T1r3 were not expressed in cell line KT-1A but were expressed in the KT-1C cells. That is, the KT-1C cells contained many cells that express T1r2 and T1r3. Consequently, cell line KT-1A and KT-1C cells can be distinguished from each other in terms of T1r2 and T1r3 expression.

Results for expression of the aforementioned marker molecules and other marker molecules are shown in Table 2 below.

**Table 2 Expression of various marker molecules in cell line KT-1A and KT-1C cells**

| Marker molecule | Expressed or not expressed | |
|---|---|---|
| | Cell line KT-1A | KT-1C cells |
| Taste bud-specific markers | | |
| Cytokeratin 8 | ++ | ++ |
| STG | + | + |

| Taste receptors and channels | | |
|---|---|---|
| T1r1 | + | + |
| T1r2 | - | ++ |
| T1r3 | - | ++ |
| T2r5 | + | - |
| T2r18 | + | + |
| T2r19 | ++ | + |
| αENaC | + | + |

| G proteins | | |
|---|---|---|
| Gustducin | - | - |
| Gαi2 | ++ | + |
| Gαi3 | ++ | + |
| Gγ13 | ++ | + |

| Undifferentiated markers | | |
|---|---|---|
| Patched 1 | ++ | ++ |
| Notch 1 | ++ | ++ |

| | | |
|---|---|---|
| Symbols in the table are defined as follows: ++: strongly expressed, +: expressed, -: not expressed. | | |

### 2-3. Immunohistochemical staining of circumvallate papilla

### (Experimental methods)

Frozen sections (10 µm) of the circumvallate papilla from the tongue of a male C57BL/6 mouse were placed on a slide which was then fixed with PBS(-) containing 4% paraformaldehyde, reacted overnight at 4°C with various anti-integrin antibodies, and stained with fluorescent-labeled secondary antibodies (Alexa 488-labeled anti-rabbit or anti-mouse IgG and Cy3-labeled anti-hamster or rat IgG).

### (Experimental results)

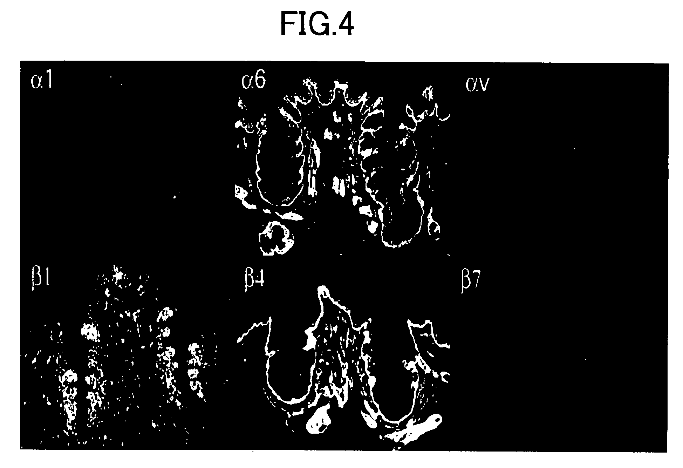

Figure 4 is an immunohistochemical staining showing the integrin expression pattern in adult mouse circumvallate papilla. As shown in Figure 4, integrin β4 and integrin α6 were strongly expressed only in the basal epithelium in mouse circumvallate papilla. Meanwhile, integrin β1 was strongly expressed not only in the basal cells but also in the taste buds.

### 2-4. Flow cytometric analysis of cell line KT-1B

### (Experimental methods)

Cell line KT-1B (2 x 10⁵ cells) was reacted for 30 minutes at 4°C with anti-integrin monoclonal antibodies (1 µg) and stained with FITC-labeled secondary antibodies, and the level of expression wasanalyzed by flow cytometry.

### (Experimental results)

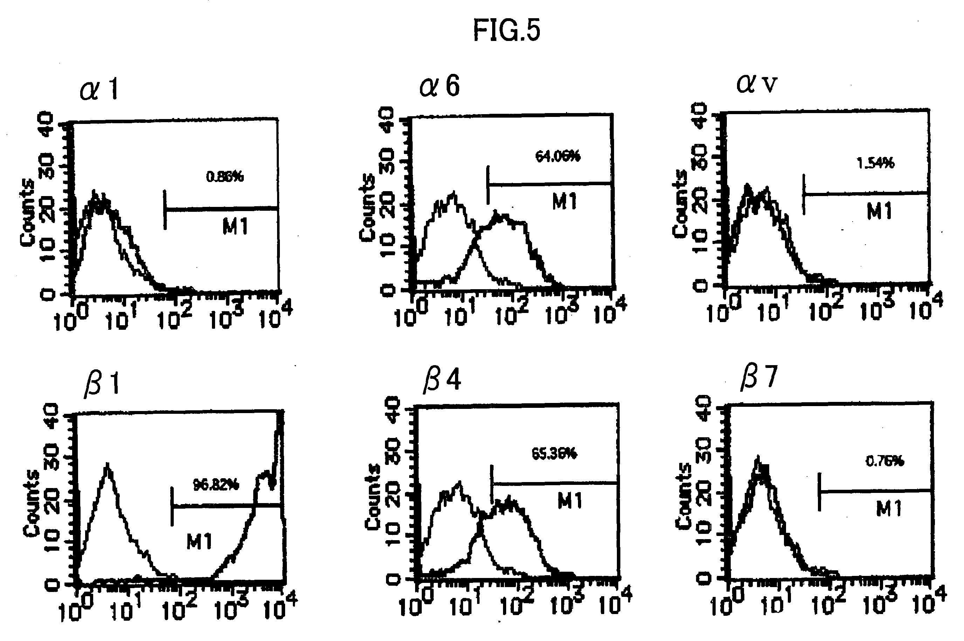

The levels of expression of various integrin molecules in cell line KT-1B are shown in Figure 5. As in the basal epithelium of the tongue near the circumvallate papilla (Figure 4), expression of α6, β1 and β4 was seen in cell line KT-1B. That is, cell line KT-1B exhibited an integrin expression pattern similar to that of the aforementioned circumvallate papilla. This indicates that both taste-bud cells and undifferentiated basal cells exist in cell line KT-1B.

### 2-5 Immuno-staining of mouse circumvallate papilla and cell line KT-1B

### (Experimental methods)

Cell line KT-1B cultured on a glass slide and mouse papilla vallata fixed for 10 minutes at room temperature with PBS(-) containing 4% paraformaldehyde as in the aforementioned Example 2-3 were reacted overnight at 4°C with anti-musashi antibodies and anti-cytokeratin 8 antibodies, and then stained with Alexa 488-labeled secondary antibodies.

### (Experimental results)

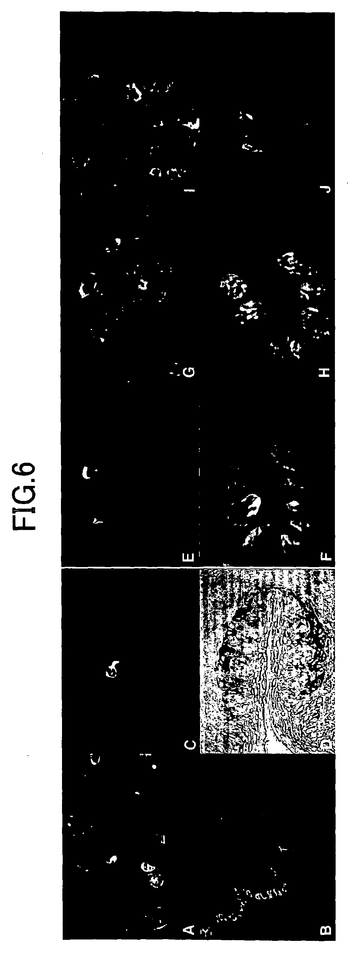

Figure 6 shows expression of undifferentiated cell-specific molecules and taste bud-specific molecules in cell line KT-1B (top) and mouse circumvallate papilla (bottom). Figures 6A and 6B show expression of the musashi protein while Figures 6E and 6F shows expression of the cytokeratin 8 protein. The scale bar is 40 µm. In Figure 6, white staining of a cell indicates expression of the various molecules.

As shown in Figures 6A and 6B, the musashi protein (marker expressed in neural stem cells) was expressed near the basal part of the circumvallate papilla, and was also strongly expressed in cell line KT-1B. As shown in Figures 6E and 6F, the taste bud-specific molecule cytokeratin 8 was also expressed in cell line KT-1B. Expression of cytokeratin 8 was seen in roughly 20 to 30% of the cells in cell line KT-1B.

### 2-6. Confirming expression of taste-related molecules by in situ hybridization of mouse circumvallate papilla and cell line KT-1B

### (Experimental methods)

cDNAs of mouse Patched 1, STG, T2r5, T2r18 and T2r19 were subcloned into pGEM-T plasmids (Promega), and these plasmids were used as the templates for preparing digoxigenin-labeled probes using a DIG RNA labeling kit (Roche). Each probe was dissolved in a hybridization solution (50% formamide, 5 x SSC, 10% Denharts, 10 mg/ml salmon sperm DNA) to a concentration of 0.5 µg/ml. Cell line KT-1B cultured on a glass slide and mouse circumvallate papilla fixed for 10 minutes at room temperature with PBS(-) containing 4% paraformaldehyde as in Example 2-3 above were each covered with hybridization liquids containing the aforementioned diluted probes, and hybridized overnight at 65°C.

After being washed with 2 x SSC (5 min. at room temperature) and 0.2 x SSC (90 minutes at 65°C) and blocked for 2 hours with 1 x tyramide blocking buffer (Dupont), these were reacted overnight at 4°C with Anti-Digoxigenin-POD (diluted 100x with blocking buffer, Roche). After being washed 3 times for 5 minutes with PBS(-) containing 0.02% Tween-20, they were reacted for 30 minutes at 37°C with Biotinyl-tyramide (diluted 50 times with tyramide reaction buffer, Dupont). After being washed 3 times for 5 minutes with PBS (-) containing 0.02% Tween-20, they were reacted with streptavidin-Alexa 488 (diluted 250x with PBS(-), Molecular Probe) and observed and photographed under a fluorescence microscope (Leica, DMIRB).

### (Experimental results)

Figure 6 shows expression of undifferentiated cell-specific molecules and taste bud-specific molecules in cell line KT-1B (top) and mouse circumvallate papilla (bottom). Figures 6C and 6D show expression of the undifferentiated cell marker Patched 1, while Figures 6G and 6H show expression of the taste bud-specific gene STG, and Figures 6I and 6J show expression of the bitter taste receptors T2r5, T2r18 and T2r19. In Figure 6, white staining indicates cells in which each molecule is expressed. However, in Figure 6D, black staining indicates cells with expression. In Figure 6, it is shown that a cell population comprising cell line KT-1B contains undifferentiated cells, cells expressing taste bud-specific molecules and cells that respond to bitter taste.

### 3. Confirmation by calcium imaging of sweet taste response ability in KT-1C cells

### (Experimental methods)

Cell line KT-1B was cultured in a 35 mm glass bottomed dish (Matsunami Glass), and once 50-70% confluence had been reached the differentiation-inducing treatment described in Example 1-3 was applied for 3 to 7 days to obtain KT-1C cells.

KT-1C cells which had been washed twice in PBS(-) were incubated for 30 minutes at room temperature with 1 ml of imaging buffer (NaHCO₃ 22 mM, KCl 5 mM, NaH₂PO₄ 1.25 mM, glucose 10 mM, NaCl 124 mM) to which 5 µl of Fura-2-AM (1 mM dissolved in DMSO, Molecular Probe) and 5 µl of Cremophor (Nacalai Tesque) had been added, incorporating Fura-2-AM into the cells. They were then washed twice with imaging buffer to remove any Fura-2-AM that had not been incorporated into the cells.

Next the dish was filled with 1 ml of imaging buffer to which 1 mMMgCl₂ and 2 mMCaCl₂ had been added, and the artificial sweetener Acesulfame K (Fluka), which is known to be a ligand for sweet taste receptors T1r2 and T1r3, was added to a final concentration of 5 mM. It is known that when cells with sweet taste response ability (in other words, cells in which the sweet taste receptors T1r2 and T1r3 are functionally expressed) respond to Acesulfame K (sweet substance) , calcium flows into the cytoplasm and the concentration of calcium in the cytoplasm rises. Therefore, fluorescence (490 nm) emitted by the Fura-2 as calcium flowed into the cytoplasm due to addition of Acesulfame K was measured over time using an inverted fluorescence microscope (Leica, DMRIB) equipped with a CCD camera (Hamamatsu Photonics) controlled by AquaCosmos software (Hamamatsu Photonics). After measurement, changes over time in florescence in the various cells were converted to changes in calcium concentration by the AquaCosmos software, and graphed.

### (Experimental results)

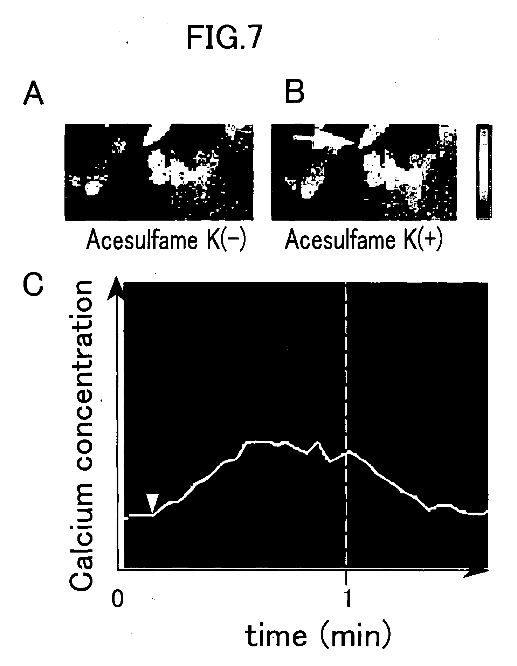

The results are shown in Figure 7. Figure 7A shows fluorescent strength before addition of Acesulfame K, while Figure 7B shows fluorescent strength 0.5 minutes after addition of Acesulfame K. The color bar at the right of Figure 7B indicates stronger fluorescence moving from blue to red.

Comparing the cells indicated by the arrow in Figure 7B as they appear in Figures 7A and 7B, the fluorescent strength changed from green to yellow and red when Acesulfame K was added. This shows that addition of Acesulfame K resulted in a flow of calcium into the KT-1C cells and a rise in intercellular calcium concentration.

Figure 7C shows changes over time in calcium concentration in the cells indicated by the arrow in Figure 7B. In Figure 7C, the horizontal axis indicates time (minutes) from the start of measurement and the vertical axis indicates the intercellular calcium concentration. The point indicated by the arrow is the point at which Acesulfame K was added. It can be seen from Figure 7C as well that addition of Acesulfame K triggered a flow of calcium into the KT-1C cells, resulting in a rise in intercellular calcium concentration.

It can thus be seen that KT-1C cells functionally express the sweet taste receptors T1r2 and T1r3, and have sweet taste response ability. This indicates that KT-1C cells can be used as a taste sensor for sensing sweet taste, and can be used to search for substitute sweeteners and sweetness-enhancing substances.

The cells with sweet taste response ability of this example can be isolated by using the aforementioned rise in intercellular calcium concentration due to addition of Acesulfame K as the indicator in selecting KT-1C cells obtained by treating cell line KT-1B (deposited as FERM BP-8347) with the MCDB basic medium C described in Example 1-3.

### 4. Hormone regulation of taste receptor expression using cell line KT-1B

### (Experimental methods)

While being cultured in the presence of hydrocortisone, cell line KT-1B constantly expresses the amiloride-sensitive sodium channel alpha subunit (αENaC), which might be involved in salt taste response.

Therefore, cell line KT-1B was treated for 6 days with the hormone progesterone (0.1 µM), aldosterone (1 µM) or hydrocortisone (0.2 µM), and expression of the salt sensing channel αENaC was investigated.

RNA was extracted with Trizol solution from the treated cell line KT-1B, and cDNA was synthesized on a scale of 40 µl using the resulting 2µg of RNA and a Super Script First Strand cDNA synthesis kit. Using 1 µl of this cDNA as the template and primers specific to salt taste channel αENaC (0.2 µM each, Seq. Nos. 13 and 14), 2.5 units of Takara Taq were added and a PCR reaction consisting of denaturation at 95°C for 5 minutes followed by 35 cycles of 1 minute at 95°C, 1 minute at 58°C and 2 minutes at 72°C was performed in a 25 µl system. 8 µl of the PCR reaction liquid was electrophoresed on a 1.5% agarose gel, stained with ethidium bromide and photographed.

### (Experimental results)

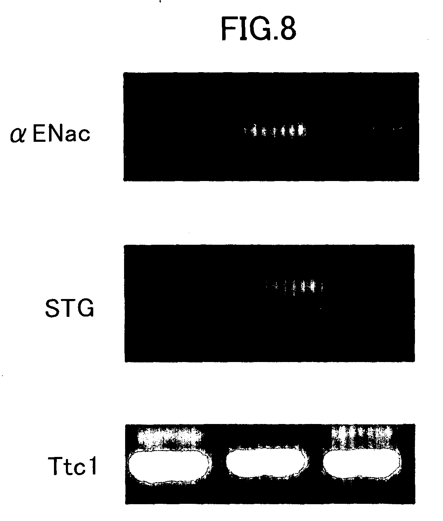

Results for detection of taste receptor mRNA in cell lines KT-1B treated with various hormones (lane 1: progesterone, lane 2 : hydrocortisone, lane 3: aldosterone) are shown in Figure 8.

As shown in Figure 8, the amount of expression of the salt taste channel αENaC was greater in the cells treated with aldosterone or hydrocortisone than in the cells treated with progesterone.

### 5. Confirmation by sodium imaging of salt taste response ability of KT-1C cells

### (Experimental methods)

KT-1C cells induced as in Example 3 were washed in 2 stages with PBS (-) , and incubated for 3 minutes at room temperature with 1 ml Na imaging buffer (NaHCO₃ 22 mM, KCl 5 mM, NaH₂PO₄ 1.25 mM, glucose 10 mM, N-methyl D-glucamine 124 mM) to which 1 µl CoroNa-Red (Molecular Probe Co.) had been added, incorporating CoroNa-Red into the cells. Next they were washed twice with Na imaging buffer to remove any CoroNa-Red that had not been incorporated into the cells.

Next, the dish was filled with 1 mL of imaging buffer to which 1 mM MgCl₂ and 2 mM CaCl₂ had been added, and NaCl, which is known to be a salt taste channel ligand, was then added thereto to a final concentration of 50 mM. It is known that when cells with salt taste response ability (in other words, cells in which the salt sensing channel αENaC is functionally expressed) respond to NaCl (salty substance), sodium flows into the cytoplasm, and the concentration of sodium in the cytoplasm rises. Therefore, the fluorescence (580 nm) produced by CoroNa-Red when sodium flowed into the cytoplasm due to addition of NaCl was measured over time using an inverted fluorescence microscope (Leica, DMRIB) equipped with a CCD camera (Hamamatsu Photonics) controlled by AquaCosmos software (Hamamatsu Photonics). After measurement, changes over time in florescence in the various cells were converted to changes in sodium concentration using the AquaCosmos software, and graphed.

### (Experimental results)

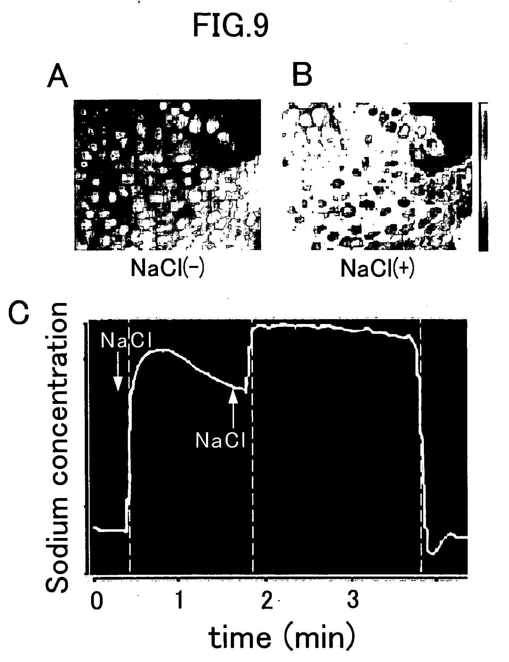

The results are shown in Figure 9. Figure 9A shows florescent strengthbefore addition of NaCl, while Figure 9B shows fluorescent strength 0.5 minutes after addition of NaCl. The color bar at the right of Figure 9B indicates stronger fluorescence moving from blue to red.

Comparing Figures 9A and 9B, fluorescent strength changed from green to yellow and red due to NaCl addition. This shows that addition of NaCl resulted in a flow of sodium into the KT-1C cells and a rise in intercellular sodium concentration.

Figure 9C shows changes over time in the sodium concentration of the cells indicated by the arrow in Figure 9B. In Figure 9C, the horizontal axis indicates time (minutes) from the start of measurement and the vertical axis indicates the intercellular sodium concentration. The point indicated by the downward-pointing arrow is the point at which 50 mM NaCl was added, while the point indicated by the upward-pointing arrow is the point at which 150 mM NaCl was added. It can be seen from Figure 9C that addition of NaCl triggered a flow of sodium into the KT-1C cells, resulting in a rise intercellular sodium concentration. The intercellular sodium concentration was also affected by the concentration of added NaCl.

It can thus be seen that KT-1C cells functionally express the salt sensing channel αENaC, and have ability to respond salt taste. This indicates that KT-1C cells can be used as a taste sensor for sensing salt tastes, and can be used to search for salt substitutes and salt taste-enhancing substances.

The cells with salt taste response ability of this example can be isolated by using the aforementioned rise in intercellular sodium concentration due to addition of NaCl as the indicator in selecting KT-1C cells obtained by treating cell line KT-1B (deposited as FERM BP-8347) with the MCDB basic medium C described in Example 1-3.

### 6. Confirmation by calcium imaging of bitter taste response ability in cell line KT-1B

### (Experimental methods)

Cell line KT-1B was cultured in a 35 mm glass bottomed dish (Matsunami Glass), and once 50-70% confluence had been reached, KT-1B cells which had been washed twice in PBS(-) were incubated for 30 minutes at room temperature with 1 ml of imaging buffer (NaHCO₃ 22 mM, KCl 5 mM, NaH₂PO₄ 1.25 mM, glucose 10 mM, NaCl 124 mM) to which 5 µl of Fluo-3-AM (1 mM solution dissolved in DMSO, Dojin Chemical) and 5 µl of Cremophor (Nacalai Tesque) had been added, incorporating the Fluo-3-AM into the cells. They were then washed twice with imaging buffer to remove any Fluo-3-AM that had not been incorporated into the cells.

Next the dish was filled with 1 ml of imaging buffer to which 1 mM MgCl₂ and 2 mM CaCl₂ had been added, and cycloheximide, which is known to be a ligand for the bitter taste receptor T2r5, was added to a final concentration of 1mM. It is known that when cells with bitter taste response ability (in other words, cells in which the bitter taste receptor T2r5 is functionally expressed) respond to cycloheximide (bitter taste substance), calcium flows into the cytoplasm and the concentration of calcium in the cytoplasm rises. Therefore, fluorescence (490 nm) emitted by the Fluo-3 as calcium flowed into the cytoplasm due to addition of cycloheximide was measured over time using an inverted fluorescence microscope (Leica, DMIRB) equipped with a CCD camera (Hamamatsu Photonics) controlled by AquaCosmos software (Hamamatsu Photonics). After measurement, changes over time in florescence in the various cells were converted to changes in calcium concentration using the AquaCosmos software, and graphed.

The cells with bitter taste response ability of this example can be isolated by using the aforementioned rise in intercellular calcium concentration due to addition of cycloheximide as the indicator in selecting cell line KT-1B (deposited as FERM BP-8347).

### INDUSTRIAL APPLICABILITY

Using the cell line KT-1 of the present invention, it is possible to reproducibly measure a specific taste response and construct a system for identifying a factor which induces taste cell differentiation.

## Claims

1. A cell line KT-1 isolated from mouse tongue epithelium using high-level expression of integrin β1 as the indicator and established by a long-term culture, and which expresses a taste receptor.

2. A cell line KT-1 established by a long-term culture of the cell line KT-1 according to Claim 1 in the following low-serum medium.
| | |
|---|---|
| Calcium | Final concentration5 µg/ml |
| Epidermal growth factor | Final concentration 10 ng/ml |
| Basic fibroblast growth factor | Final concentration 10 ng/ml |
| Insulin | Final concentration 5 ng/ml |
| Transferrin | Final concentration 10 ng/ml |
| Hydrocortisone | Final concentration 0.2 µM |
| Ethanolamine | Final concentration 0.5 µM |
| Phosphoethanolamine | Final concentration 0.5 µM |
| Heparin | Final concentration20 µg/ml |
| Gentamicin | Final concentration 10 mg/ml |
| Amphotericin B | Final concentration 500 ng/ml |
| Penicillin | Final concentration 50 U/ml |
| Streptomycin | Final concentration 50 mg/ml |
| Calcium-chelated fetal bovine serum | Final concentration 0.25% |

3. The cell line KT-1 according to Claim 2, deposited under Accession Number FERM BP-8347.

4. A KT-1 cell obtained by culturing the cell line KT-1 according to Claim 2 or 3 in the following differentiation-inducing medium
| | |
|---|---|
| Calcium | Final concentration 10 µg/ml |
| Epidermal growth factor | Final concentration1 ng/ml |
| Keratinocyte growth factor | Final concentrationl ng/ml |
| Insulin-like growth factor | Final concentration 5 ng/ml |
| Transferrin | Final concentration 100 ng/ml |
| Hydrocortisone | Final concentration 0.2 µM |
| Aldosterone | Final concentration 10 nM |
| Ethanolamine | Final concentration 0.5 µM |
| Phosphoethanolamine | Final concentration 0.5 µM |
| Calcitriol | Final concentration 10 nM |
| 9-cis retinoic acid | Final concentration 0.8 µM |
| Glutathione | Final concentration 200 µM |
| Cysteine | Final concentration 50 µM |
| Tyrosine | Final concentration 2.72 µg/ml |
| Phenylalanine | Final concentration 4.95 µg/ml |
| Heparin | Final concentration 20 µg/ml |
| Gentamicin | Final concentration 10 mg/ml |
| Amphotericin B | Final concentration500 ng/ml |
| Penicillin | Final concentration 50 U/ml |
| Streptomycin | Final concentration 50 mg/ml |

5. The KT-1 cell according to Claim 4, which functionally expresses a taste receptor.

6. A taste sensor comprising the cell line KT-1 according to any of Claims 1 through 3 or the KT-1 cell according to Claim 4 or 5.

7. The use of a cell line KT-1 as a taste sensor, wherein the cell line KT-1 according to any of Claims 1 through 3 or the KT-1 cell according to Claim 4 or 5 is used to distinguish a taste substance received by a taste receptor.

8. The use according to 7, wherein the taste receptor is a sweet taste receptor, bitter taste receptor, salt taste channel or amino acid receptor.

9. A cell line KT-1 transformed by cDNA transfection of a taste receptor which is not expressed in cell line KT-1, and a ligand for which is known.

10. The use of the transformed cell line KT-1 according to Claim 9 as a cell for detecting a specific taste which is received by the taste receptor.

11. A cell line KT-1 transformed by cDNA trasnfection of a taste receptor which is not expressed in cell line KT-1, and ligand for which is unknown.

12. A method for searching for an unknown ligand by using the transformed cell line KT-1 according to Claim 11.

13. The use of the cell line KT-1 according to any of Claims 1 through 3 or the KT-1 cell according to Claim 4 or 5 for searching for a factor which induces differentiation of taste cells, a substance which controls expression of taste receptors, a novel taste substance, a taste-modifying substance and a substance which aids in taste-suppressed state.

14. A method of searching for a taste receptor for a taste substance by using the taste substance and the cell line KT-1 according to any of Claims 1 through 3 or the KT-1 cell according to Claim 4 or 5.

15. The method according to Claim 14, wherein the taste receptor is a salt sensing channel.
